# EUROPEAN PATENT APPLICATION

(11) **EP 3 733 708 A1**
(43) Date of publication of application: **04.11.2020**
(21) Application number: 19172399.8
(22) Date of filing: 02.05.2019
(51) Int. Cl.: C07K 16/28, A61P 35/00

(54) **IMMUNE CHECKPOINT MOLECULE INHIBITOR**

(71) Applicant: Randox Laboratories Ltd, Crumlin, Antrim BT29 4QY (GB)
(72) Inventor: Dunlop, Marshall, Crumlin Antrim BT29 4QY (GB); Schon, Shirley, Crumlin Antrim BT29 4QY (GB); Lamont, John, Crumlin Antrim BT29 4QY (GB); Fitzgerald, Stephen, Crumlin Antrim BT29 4QY (GB)
(74) Representative: Gill Jennings & Every LLP

(57) **Abstract**

The present invention provides a bispecific binding protein comprising a first and second functional moiety, each having binding affinity for a different epitope located on the same immune checkpoint molecule, for example, PD-1, PD-L1, CTLA-4 and CD47. Also provided is a pharmaceutical composition comprising the bispecific binding protein of the invention for use in therapy, particularly for use in the treatment of cancer and infectious disease.

## Description

### Field of Invention

The present invention relates to binding molecules with specificity for immune checkpoint molecules. The present invention also relates to pharmaceutical compositions comprising the binding molecules for use in the treatment of cancer and infectious disease.

### Background

Immune checkpoint molecules are molecules which regulate the activity of the immune system. The ability to self-regulate the activity of immune cell types is crucial in host survival; protecting the host against excessive tissue damage and preventing the development of autoimmune diseases; a group of diseases characterised by the immune system indiscriminately attacking the body's cells (opposed to cells of foreign origin).

Immune checkpoint molecules may be stimulatory or inhibitory, allowing the creation of a highly regulated homeostatic system in which immune cell type activity can be controlled. As a primary mediator of immune effector function, activated T-cells are one type of immune cell which are subjected to such control via the expression of multiple inhibitory immune checkpoint molecules. Examples of such molecules include programmed cell death protein-1 (PD-1), cytotoxic T-lymphocyte-associated protein 4 (CTLA-4) and lymphocyte-activation gene 3 (LAG-3).

Programmed Cell Death Protein 1, (PD-1 or CD279), a 55-kD type 1 transmembrane protein, is a member of the CD28 family of T cell co-stimulatory receptors that include immunoglobulin superfamily member CD28, CTLA-4, inducible co-stimulator (ICOS), and BTLA. PD-1 is highly expressed on activated T cells and B cells. PD-1 expression can also be detected on memory T-cell subsets with variable levels of expression. Two ligands specific for PD-1 have been identified: programmed death- ligand 1 (PD-L1, also known as B7-H1 or CD274) and PD-L2 (also known as B7-DC or CD273). PD-L1 and PD-L2 have been shown to down-regulate T cell activation upon binding to PD-1 in both mouse and human systems (Okazaki et al., Int Immunol., 2007; 19: 813-824). The interaction of PD-1 with its ligands, PD-L1 and PD-L2, which are expressed on antigen-presenting cells (APCs) and dendritic cells (DCs), transmits negative regulatory stimuli to down-modulate the activated T cell immune response. Activation of either of these ligands leads to dephosphorylation and inactivation of the T-cell kinase ZAP70 and the recruitment of SHP2. SHP2 directly dephosphorylates PI3K, inhibiting the downstream activation of Akt and ultimately leads to a reduction in inflammatory cytokines and cell survival proteins, triggering apoptotic pathways and leading to the destruction of the T-cell. Blockade of PD-1 suppresses this negative signal and amplifies T cell responses.

CTLA-4 is a transmembrane glycoprotein that is a CD28 homologue, the latter of which is an immune co-stimulatory protein. Following T-cell activation, CTLA-4 is transported to the surface where it competes with CD28 for the same ligands as on the antigen-presenting cells (APCs), resulting in suppression of CD28 and subsequent suppression of T-cell activation and proliferation.

Similar to both PD-1 and CTLA-4, LAG-3 is known to be critical for dampening overt T-cell immune responses. The main ligand known to bind to LAG-3 is major histocompatibility complex (MHC) class II, found primarily on professional APCs. It is known that LAG-3 specifically inhibits CD8⁺ effector T-cell functions and can enhance the activity of Tregs (regulatory T-cells). It has recently been revealed that PD-1 and LAG-3 are extensively co-expressed by tumour-infiltrating T cells in mice, and that combined blockade of PD-1 and LAG-3 provokes potent synergistic antitumor immune responses in mouse models of cancer.

CD47 is a ubiquitously expressed glycoprotein of the immunoglobulin superfamily that plays a critical role in self-recognition. It is known to have an essential role as a negative checkpoint for innate immunity and subsequent adaptive immunity. CD47 can interact with signal regulatory protein-alpha (SIRPα) expressed on myeloid cells, resulting in phosphorylation of the SIRPα cytoplasmic immunoreceptor tyrosine-based inhibition motifs and recruitment of Src homology 2 domain-containing tyrosine phosphates. This signalling pathway ultimately results in the delivery of an anti-phagocytic signal.

Despite the purpose of this regulatory system being to protect the host, numerous scientific studies have demonstrated that the tumour microenvironment is capable of hijacking this system. For example, numerous studies indicate that the cancer microenvironment manipulates the PD-L1-/PD-1 signalling pathway and that induction of PD-L1 expression is associated with inhibition of immune responses against cancer, thus permitting cancer progression and metastasis. The PD-L1/PD-1 signalling pathway is a primary mechanism of cancer immune evasion for several reasons. First, and most importantly, this pathway is involved in negative regulation of immune responses of activated T effector cells, found in the periphery. Second, PD-L1 is up-regulated in cancer microenvironments, while PD-1 is also up-regulated on activated tumour infiltrating T cells, thus possibly potentiating a vicious cycle of inhibition. Third, this pathway is intricately involved in both innate and adaptive immune regulation through bi-directional signalling. These factors make the PD-1/PD-L1 complex a central point through which cancer can manipulate immune responses and promote its own progression. As a result, the tumour is able to activate inhibitory immune checkpoint molecule pathways, resulting in a suppressed immune system and the continued unimpeded growth of cancerous cells. Similarly, growing evidence in the scientific community points toward the exploitation of immune checkpoints in infectious disease, for example, HIV, tuberculosis and malaria. Consequently, the prevention of this interaction between immune cells and tumour cells has been a focus of therapeutic intervention in the cancer field for quite some time and now represents a growing area of interest in the infectious disease field.

A number of monoclonal antibodies have been developed to target either PD-1 or PD-L1; these include Pembrolizumab (Keytruda), Nivolumab (Opdivo), Cemiplimab (Libtayo), Atezolizumba (Tecentriq) and Durvalumab (Imfinzi). These drugs have been shown to be beneficial in treating a number of different cancers, including melanoma of the skin, non-small cell lung cancer and Hodgkin lymphoma. Similarly, drugs targeting CTLA-4 and LAG-3 have also been developed for use against a variety of cancers. The anti-CTLA-4 monoclonal antibody Ipilimumab (Yervoy) is an example of such a drug. Anti-CTLA-4 monoclonal antibodies are powerful checkpoint inhibitors which remove 'the brake' from both naive and antigen-experienced cells. Therapy enhances the antitumor function of CD8+ T cells, increases the ratio of CD8+ T cells to Foxp3+ T regulatory cells, and inhibits the suppressive function of T regulatory cells. The major drawback to anti-CTLA-4 monoclonal antibody therapy is the generation of autoimmune toxicities due to off-target effects of an over-exuberant immune system which has lost the ability to turn itself down. It has been reported that up to 25% of patients treated with ipilimumab developed serious grade 3-4 adverse events/autoimmune-type side effects including dermatitis, enterocolitis, hepatitis, endocrinopathies (including hypophysitis, thyroiditis, and adrenalitis), arthritis, uveitis, nephritis, and aseptic meningitis.

What is lacking in the prior art is a therapy with minimal side-effects whilst displaying desirable blocking capabilities. Given the current systemic reduction in immune self-tolerance it would be beneficial for a more targeted delivery of such immune checkpoint inhibitors to improve the side-effects associated with this type of therapeutic intervention.

### Summary of Invention

The inventors of the present invention have found that a bispecific binding protein, targeting different epitopes on the surface of the same immune checkpoint molecule, for example PD-1, PD-L1, CTLA-4 and CD47, results in an unexpected synergistic blocking effect as compared to targeting the same epitopes with a mixture of monospecific binding constituents which have not been linked together in a single bispecific binding protein.

In a first aspect, the present invention provides for a bispecific binding protein comprising a first functional moiety and a second functional moiety each having binding affinity for an immune checkpoint molecule, wherein the first functional moiety and the second functional moiety are connected, and wherein the first functional moiety and the second functional moiety bind to different discrete epitopes on the surface of the same immune checkpoint molecule.

In a second aspect, the present invention provides for a nucleic acid encoding the bispecific binding protein as defined in the first aspect of the present invention.

In a third aspect, the present invention provides for an expression vector comprising the nucleic acid as defined in the second aspect of the present invention.

In a fourth aspect, the present invention provides for a host cell or cell free expression system comprising the expression vector as defined in the third aspect of the present invention.

In a fifth aspect, the present invention provides for a pharmaceutical composition comprising the bispecific binding protein, nucleic acid, vector or host cell disclosed herein and medical uses thereof.

### Description of Figures

The invention is described with reference to the accompany drawing, wherein;
**Figure 1** is a graph showing the blocking capability of a range of bispecific constructs comprising the lead clones 9E3 and 3H4, linked with a peptide linker of either 17 aa or 30 aa in both directions, and compared against the blocking capability of either clone alone and against the commercially available anti-PD1 drug Nivolumab.
**Figure 2** is a graph showing the blocking capability of the bispecific construct GS388 as compared to a simple mix of its unlinked constituent binding domains.
**Figure 3** is a graph showing the relative apparent binding affinities of bispecific clones directed to PD-1 as compared to their single domain antibody constituents 9E3 and 3H4.
**Figure 4** is a schematic demonstrating the disruption of the binding interaction between PD-1 and a biotinylated recombinant human PD-L1Fc, by introducing a range of concentrations of the blocking antibodies or Nivolumab.
**Figure 5** is a schematic demonstrating the apparent affinities of the bispecific clone GS388 and its parental sdAb constituent clones to PD-1 antigen, detected by an anti-cMyc-tag HRP detector conjugate.
**Figure 6** is a schematic showing how the first functional moiety and the second functional moiety of the bispecific binding protein may be connected, non-covalently, by paired fusion tags, Specifically, the fusion tag is the C_{H}2 and C_{H}3 domains of an antibody Fc domain.
**Figure 7** is a schematic demonstrating how an Fc domain can be used to generate an IgG-like homodimer with two arms, each arm being capable of binding two epitopes on the same target antigen.

### Detailed Description

In a first aspect, the present invention provides for a bispecific binding protein comprising a first functional moiety and a second functional moiety each having binding affinity for an immune checkpoint molecule, wherein the first functional moiety and the second functional moiety are connected, and wherein the first functional moiety and the second functional moiety bind to different discrete epitopes on the surface of the same immune checkpoint molecule.

The term 'bispecific binding protein' refers to an artificial protein that combines the specificities of two antibodies, or antibody-like binding proteins, into one molecule which can simultaneously bind to two different antigens or epitopes. By 'first functional moiety' and 'second functional moiety' we intend the two distinct elements of the bispecific binding protein, each conferring binding specificity to a different target. In the context of the present invention, the target is two different discrete epitopes located on the same receptor antigen. The inventors have made the surprising discovery that the linkage of two antibody-like binding proteins (to create the bispecific binding protein herein disclosed) demonstrates a level of blocking of the receptor which is greater than a mixture of the two constituent antibody-like binding proteins which are not linked.

It is intended that the bispecific binding protein may act as an immune checkpoint inhibitor. An 'immune checkpoint inhibitor' is a surface protein which regulates the immune response and is typically a member of the TNF receptor, immunoglobulin or B7 superfamilies, including agents which bind to negative co-stimulatory molecules, or their ligands, such as PD-1, PD-L1, CTLA-4, LAG-3, TIGIT, A2AR, B7-H3, B7-H4, B7-H, B7-H7, BTNL2, butyrophilins, CD47, CD48, CD55, CD96, CD96R, CD112, CD112R, CD155, CD160, CD200, CD200R, CD244, CD277BTLA, IDO, KIR, MOG, NOX2, PD-L2, SIRPα, SKINT1, TMIGD2, TIM-3, VISTA, SIGLEC7 and/or their respective ligands, for example PD-L1. Preferably, the immune checkpoint molecules to be targeted include PD-1, PD-L1, CTLA-4 and CD47.

'Binding affinity' refers to the strength of the binding interaction between the bispecific binding protein and the ligand to which it is intended to bind. Binding affinity is typically measured and reported by the equilibrium dissociation constant (Kd). A skilled person in the art will recognise that the smaller the Kd value the greater the binding affinity and therefore a smaller Kd value would be desirable for a binding protein intended as a therapeutic.

The term 'epitope' refers to the site on an antigen to which a complimentary antibody may specifically bind. Specifically, this is the site of the antigen where complementary-determining regions (CDRs) of antibodies bind to. The amino acid sequence of the variable domain of an antibody contains three CDRs; CDR1, CDR2 and CDR3, the sequence of which determine the specificity of the antibody. Epitopes can be conformational epitopes or linear epitopes. It is understood that the target epitope should be one which is readily available for binding by the bispecific binding protein, to ensure an efficient interaction.

It is envisaged that the bispecific binding protein of the present invention may bind to two different discrete epitopes, located on the same immune checkpoint molecule. It is envisaged that the two different discrete epitopes will be in the vicinity of the receptor binding site of said immune checkpoint molecule. The specific epitopes to be targeted will be dependent on the target immune checkpoint molecule. The epitopes to be targeted are understood to be close enough in proximity to one another to allow simultaneous binding of the bispecific binding protein to each epitope.

The ligand interaction sites of key immune proteins are often highly conserved between mammalian species, including those typically used for antibody generation. Antibodies that display complete overlap of the binding site, thus resulting in full occlusion of the ligand, are known to be challenging to isolate in closely related species which are amenable to humanisation. The inventors of the present invention have surprisingly found that the linking of binding molecules (e.g. antibodies), which individually provide only partial blocking of a receptor, can provide an enhanced blocking entity, thus overcoming the aforementioned limitation of antibodies that display complete overlap of the binding site.

In one embodiment, the bispecific binding protein may target the following immune checkpoint molecules; PD-1, PD-L1, CTLA-4 or CD47. The present invention may also target alternative inhibitory immune checkpoint molecules or their ligands, these include, but are not limited to; A2AR, B7-H3, B7-H4, B7-H5, B7-H7, BTNL2, butyrophilins, LAG-3, CD48, CD55, CD96, CD96R, CD112, CD112R, CD155, CD160, CD200, CD200R, CD244, CD277, HVEM, BTLA, IDO, KIR, MOG, NOX2, PD-L2, SIRPα, SKINT1, TIGIT, TMIGD2, TIM-3, VISTA and SIGLEC7. In a preferred embodiment, the immune checkpoint molecule is PD-1.

The terms 'PD-1', 'PD1', 'programmed death 1', 'programmed cell death 1' are used interchangeably and taken to include variants, isoforms and species homologs of human PD-1. The complete PD-1 sequence can be found under GenBank Accession No. U64863.

The terms 'PD-L1', 'PDL1', 'programmed death ligand 1' and 'programmed cell death 1' are used interchangeably and taken to include variants, isoforms and species homologs of human PD-L1. The complete PD-L1 sequence can be found under GenBank Accession No. NP 054862.1.

The terms 'CTLA-4', 'CTLA4', 'cytotoxic T-lymphocyte-associated protein 4' are used interchangeably and taken to include variants, isoforms, and species homologs of human CTLA-4. The complete CTLA-4 sequence can be found under GenBank Accession No. L15006.

The terms 'LAG-3', 'LAG3', 'lymphocyte-activation gene 3' are used interchangeably and taken to include variants, isoforms and species homologs of human LAG-3.

The terms 'CD47', 'Cluster of Differentiation 47', 'IAP', 'integrin associated protein' are used interchangeably and taken to include variants, isoforms and species homologs of human CD47. The complete CD47 sequence can be found under GenBank Accession No. BT00697.

The bispecific binding protein displays binding affinity for non-overlapping epitopes on the immune checkpoint molecule. By 'non-overlapping' it is intended that the epitopes in question are entirely separate from one another. It is envisaged that targeting non-overlapping epitopes on the same immune checkpoint molecule may infringe the binding interface of the immune checkpoint molecule and therefore reduce an interaction between the immune checkpoint molecule and its natural ligand. It is envisaged that the non-overlapping epitopes to be targeted may be orientated in such a manner that the linker connecting the two binding moieties may span the binding interface of the immune checkpoint molecule. The latter is thought to result in an increased capacity to block receptor binding as compared to the capacity of the individual binding moieties. Accordingly, targeting epitopes which would naturally result in the blockade of such an interface due to their position on the immune checkpoint molecule should be targeted. Epitope mapping may be used to identify such epitopes.

The bispecific binding protein of the invention has the two binding moieties connected to each other in a manner that does not detrimentally impact on the function of either binding moiety. Without being bound by theory, it is believed that the connection between the two binding moieties is important for the synergistic effect observed in the blocking ability of the bispecific binding protein. Without being bound by theory, the multiplicative blocking effect may be due to steric hindrance of the connection entity across the interface of the ligand-receptor space, for example, the interaction between PD-1 and PD-L1.

The connection between the two functional moieties may be a peptide linker. The term 'peptide linker' is taken to mean a sequence of amino acids whose function is to connect the first functional moiety and the second functional moiety, thus resulting in a bispecific binding protein. Amino acids which may comprise this peptide linker include flexible residues such as glycine and serine to allow mobility of the connecting functional moieties.

It is envisaged that the peptide linker may range in number of amino acid residues. For example, the peptide linker may be 5-100 amino acid residues in length, 8-30 amino acids in length or in the most preferred embodiment, 10 to 17 amino acids in length. It is additionally envisaged that the linker should be soluble in nature and may be further optimised with certain characteristics, including bulky amino acid side groups which occupy space and help prevent access to the binding site as well as charged amino acid side groups which aid repulsion of binding ligands. It is further envisaged that the orientation of the linker may be important with regards to whether it connects N or C terminus of each functional moiety, with the N-terminus of each moiety being found closer to the binding surface.

In one embodiment the first functional moiety and the second functional moiety of the bispecific binding protein are connected by a peptide linker comprising the amino acid sequence of SEQ ID NO: 10 or an amino acid sequence having greater than 60 % homology to said amino acid sequence. For example, the peptide linker of the present invention may have at least 60 %, least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % homology to the amino acid sequence of SEQ ID NO: 10. To calculate % homology, sequence comparison software may be used, for example using the default settings on the BLAST software package. Further, also included are any of the aforementioned amino acid sequences with one or more conservative substitutions. By 'conservative substitution' we intend an amino acid replacement that changes one or more amino acids whilst resulting in a substance with similar biochemical properties. These biochemical properties may include charge, hydrophobicity and size, as would be understood by a person of skill in the art.

It is further envisaged that the first functional moiety and the second functional moiety of the bispecific binding protein may be linked, non-covalently, by paired fusion tags, preferably wherein the paired fusion tags are optionally a leucine zipper or an immunoglobulin Fc tag.

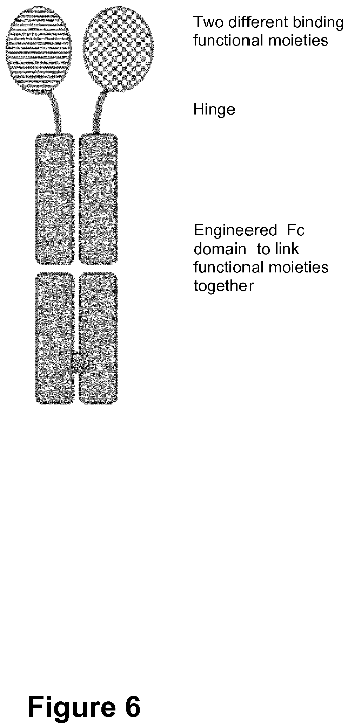

In the case of an immunoglobulin Fc tag being used to pair the first and second functional moieties, a person skilled in the art would recognise that this would require an Fc tag engineered to allow only heterodimeric pairing, at the expense of homodimeric pairing, to ensure a bispecific product. Figure 6 displays an example of how the first and second moieties may be connected by paired fusion tags, specifically wherein the fusion tag is the C_{H}2 and C_{H}3 domains of an antibody Fc domain. Engineered Fc tags can be created using 'knobs-into-holes' technology. 'Knobs-into-holes' technology refers to the process by which C_{H}3 domains are engineered to create either a 'knob' or a 'hole' in each heavy chain (Ridgway et al. (1996) Protein Engineering 9:7:617-621) This process is known to promote heterodimerisation in the production of bispecific antibodies.

The bispecific binding protein may be a bispecific antibody or a functional fragment thereof. Accordingly, the term 'antibody' as referred to herein includes whole antibodies and any antigen-binding fragments or single chains thereof.

It is envisaged that the first functional moiety and the second functional moiety of the bispecific binding protein may comprise a fragment antigen binding (Fab) fragment, a single-chain variable fragment (scFv), an affimer (or equivalent protein-binding peptide domains), or a single-domain antibody (sdAb), preferably wherein the first functional moiety and the second functional moiety is a single-domain antibody.

An 'antigen binding fragment' is a region on an antibody that binds to antigens, comprising one constant and one variable domain of each of the heavy and the light chain. In the context of the present invention, a Fab fragment is a monovalent fragment consisting of V_{L}, V_{H}, C_{L} and C_{H1} domains, the present invention also includes the use of F(ab')₂ fragments, a bivalent fragment comprising two Fab fragments linked by a disulphide bridge at the hinge region and Fv fragments, consisting of the V_{L} and V_{H} domains of a single arm of an antibody. Additionally, the present invention includes the use of scFvs, wherein the two domains of the Fv fragment, V_{L} and V_{H}, can be joined, using recombinant methods, by a synthetic linker that enable them to become a single protein chain in which the V_{L} and V_{H} regions pair to form monovalent molecules (Bird et al. (1988) Science 242:423-426; and Huston et. al. (1988) Proc. Natl. Acad Sci. USA 85:5879-5883). The order of the domains in the resulting scFv may be either V_{H}-linker-V_{L} or V_{L}-linker-V_{H}. The present invention further includes the use of a single-domain antibody (Ward et al., (1989) Nature 341:544-546), also known as VHHs, that can form a functional antigen binding site without interaction with another variable domain, for example the V_{H}-V_{L} interaction present in an immunoglobulin antibody.

The types of antibodies herein disclosed may include the use of humanised antibodies and preferably, human antibodies. It is understood that the type of antibody employed will be dependent on the end user, i.e. human or non-human.

The term 'human antibody' as used herein, is intended to include antibodies having variable regions in which both the framework and the CDR regions are derived from human germline immunoglobulin sequences.

The term 'humanised antibody' as used herein, is intended to include antibodies in which CDR sequences derived from the germline of another mammalian species, such as a mouse, have been grafted onto human framework sequences. Alternatively, the framework regions of a non-human antibody may be mutated to increase its homology to a human antibody framework.

In a further, separate embodiment, it is envisaged that an Fc tag can be used to link the bispecific binding protein herein described to another bispecific binding protein.

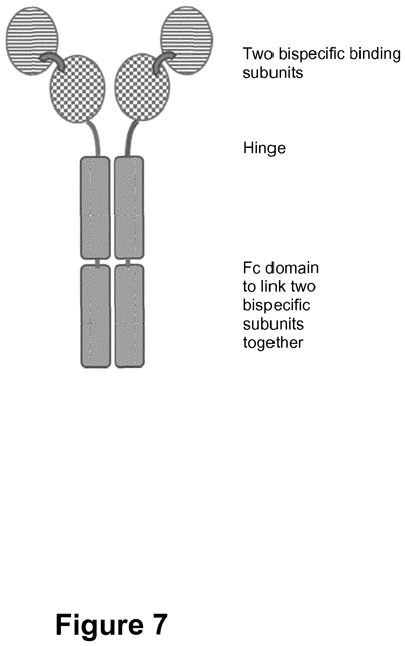

The use of an Fc tag in this manner results in the creation of an IgG-like homodimer with two arms, each arm being capable of binding two epitopes on the same target antigen. This results in an IgG-like homodimer as displayed in Figure 7.

In one embodiment, the bispecific binding protein further comprises an Fc fusion tag comprising the amino acid sequence of SEQ ID NO: 12 to generate an Ig-like homodimer with two arms (SEQ ID NO: 11). The Fc fusion tag may comprise an amino acid sequence having greater than 60 % homology to SEQ ID NO: 12. For example, the Fc fusion tag of the present invention may have at least 60 %, least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % homology to the amino acid sequences of SEQ ID NO: 12. To calculate % homology, sequence comparison software may be used, for example using the default settings on the BLAST software package. Further, also included are any of the aforementioned amino acid sequences with one or more conservative substitutions. A skilled person in the art will recognise that the addition of an Fc fusion tag confers a number of additional advantageous properties, for example, significant extension of the plasma half-life, improvement in the solubility and the stability of molecule, and allows for a cost-effective purification process. In addition, the presence of an Fc domain will encode antibody effector functions such as Antibody-Dependent Cell-Mediated Cytotoxicity (ADCC), and Complement Dependent Cytotoxicity (CDC) which may be desirable for specific applications.

The present invention may further include the use of affirmers or other protein-binding peptide domains. Affimer molecules are small proteins that bind to target molecules with similar specificity and affinity to that of antibodies. These binding proteins are designed to mimic the recognition characteristics of antibodies whilst displaying numerous advantageous properties; high thermostability, low molecular weight and resistance to extreme pH, freeze-thaw cycles and lyophilisation.

The aforementioned antibody fragments and affirmer molecules are obtained using conventional techniques known to a skilled person in the art, and the fragments screened for utility in the same manner as an intact antibody.

In one embodiment, the bispecific binding protein may comprise the amino acid sequence of SEQ ID NO:1 or SEQ ID NO:11, or an amino acid sequence having greater than 60 % homology to said amino acid sequence. For example, the bispecific binding protein may have at least 60 %, least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % homology to the amino acid sequences of SEQ ID NO:1 or of SEQ ID NO: 11. To calculate % homology, sequence comparison software may be used, for example using the default settings on the BLAST software package. Further, also included are any of the aforementioned amino acid sequences with one or more conservative substitutions.

The first functional moiety may comprise the amino acid sequence of SEQ ID NO: 2 and the second functional moiety may comprise the amino acid sequence of SEQ ID NO: 3, or an amino acid sequence having greater than 60 % homology to said amino acid sequence. For example, the first and second functional moieties of the present invention may have at least 60 %, least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % homology to the amino acid sequences of SEQ ID NO: 2 and SEQ ID NO: 3. To calculate % homology, sequence comparison software may be used, for example using the default settings on the BLAST software package. Further, also included are any of the aforementioned amino acid sequences with one or more conservative substitutions. The first and second functional moieties are interchangeable. For example, both the bispecific binding proteins produced by SEQ ID NO: 2-linker-SEQ ID NO: 3 and SEQ ID NO: 3-linker-SEQ ID NO: 2 would be effective for the purpose of the invention herein disclosed. A therapeutic product can also be envisaged which contains only one of SEQ ID NO: 2 or SEQ ID NO: 3.

Yet further, it is envisaged that the first functional moiety of the bispecific binding protein may comprise the following CDR amino acid sequences; SEQ ID NO: 4, SEQ ID NO: 5 and SEQ ID NO: 6. The second functional moiety of the bispecific protein may comprise the following CDR amino acid sequences; SEQ ID NO: 7, SEQ ID NO: 8 and SEQ ID NO: 9. Alternatively, the functional moieties may comprise CDR amino acid sequences having greater than 60 % homology to said amino acid sequence. For example, the first and second functional moieties of the present invention may have at least 60 %, least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % homology to the amino acid sequences of any of SEQ ID NOs: 4-9. To calculate % homology, sequence comparison software may be used, for example using the default settings on the BLAST software package. Further, also included are any of the aforementioned amino acid sequences with one or more conservative substitutions. For the bispecific binding protein to be functional, the CDRs are required to be in the following order within the amino acid sequence; CDR 1 (SEQ ID NO: 4), CDR 2 (SEQ ID NO: 5) and CDR 3 (SEQ ID NO: 6).

It is envisaged that the individual moieties (SEQ ID NO: 2 and SEQ ID NO: 3) of the bispecific binding protein may be useful in diagnostic applications and be used as probes for the detection of immune checkpoint molecules. Alternatively, the bispecific binding protein (SEQ ID NO: 1 and SEQ ID NO: 11) could be used as a probe to ensure specificity. Accordingly, in a separate aspect there is a composition comprising a molecule having the amino acid sequence of SEQ ID NO: 2 or SEQ ID NO: 3. There is also the use of either or both molecules in a diagnostic assay or as a binding protein in an assay. In yet a further separate aspect there is a composition comprising a molecule having the amino acid sequence of SEQ ID NO: 1 or SEQ ID NO: 11 . There is also the use of either or both molecules in a diagnostic assay or as a binding protein in an assay.

It is envisaged that the format of the bispecific binding protein herein disclosed, for example, sdAb-linker-sdAb, may be further adapted to include the use of trispecific binding molecules. For a trispecific binding molecule the format may be the following; sdAb-linker-sdAb-linker-sdAb.

In a further aspect, the present invention provides for nucleic acids/nucleotide sequences encoding all of the amino acid sequences as defined herein. The nucleic acids/nucleotide sequences may, for example, be DNA or RNA and may or may not contain intronic sequences. Yet further, the present invention provides a nucleic acid that hybridises to the nucleic acid of the present invention under low and/or high stringency conditions. By 'high stringency conditions' we include high temperature and low salt content in hybridisation buffers. By 'low stringency conditions' we include lower temperatures and high salt content in hybridisation buffers.

In a further aspect, the present invention provides an expression vector comprising a nucleic acid according to the invention. The expression vector may be used to manufacture the amino acid sequences of the invention *in vitro* (for subsequent formulation and delivery) or indeed to be delivered direct to the patient to be treated where the expression of the resulting bispecific binding protein of the invention will occur in the subject themselves.

By 'subject' we intend any human or non-human animal. The term 'non-human animal' includes all vertebrates, for example, both mammals and non-mammals, such as non-human primates, sheep, dogs, cats, horses, goats, cows, chickens, amphibians, reptiles and the like. Therefore, the present invention is intended for use in veterinary applications in addition for use in human subjects.

The vector may be any suitable vector for *ex vivo* or *in vivo* expression. The vector may be a vector suitable for use in cancer or in infectious disease. The vector may alternatively be any vector suitable for use for expression in bacteria, mammalian cell lines, plants, yeasts, protozoa or cell free expression systems. Suitable examples of vectors according to the invention are viruses or plasmids. Examples of appropriate viruses include, but are not limited to, retroviruses, adenoviruses, poxviruses and the herpes simplex virus. Preferably, oncolytic viruses may be used; viruses which preferentially infect and kill cancer cells, in the process releasing tumour cell antigens and, working in tandem with the bispecific binding protein, to generate an immune response against the target cell, for example, a tumour cell. Using virally transduced binding proteins, the product may be delivered directly to the target, for example, the tumour, and therefore exert its primary influence in the immediate vicinity. Further, when it leaches out of such an environment, it will be rapidly filtered through the kidneys and cleared from the circulation, minimising unnecessary exposure and reducing any associated toxicity. Additionally, single-domain antibodies have the ability to be co-transfected and expressed in combination due to the absence of light chains. It is the presence of light chains which typically confound the ability of a cell to correctly pair the heavy and light chains of more than one traditional antibody sequence in the same cell.

The promoter used to achieve activation of said nucleotide sequence may be selected from the group comprising: lentivirus, adeno-associated virus (AAV), cytomegalovirus (CMV), Rous sarcoma virus (RSV), elongation factor 1 alpha (EF1α), Tet-regulatable promoter, platelet-derived growth factor beta (PDGFβ), murine leukemia virus long terminal repeat (Mo-MLV-L TR), phosphoglycerate kinase (PGK), Mx1 and calmodulin-dependent protein kinase II (CaMK II). Other suitable promoters or vectors will be readily known to those skilled in the art.

In a further aspect, the present invention provides a host cell or cell free expression system comprising an expression vector according to the invention. The host cell may be mammalian, bacterial, yeast, plant or insect derived. Preferably, the host cell is mammalian to encourage the assembly and secretion of a properly folded and immunologically active antibody. 'Mammalian' is taken to include cells from human, feline, porcine, simian, canine, murine and rat origin. Chinese hamster ovary (CHO) cells, NS0 myeloma cells, COS cells and SP2 cells may be utilised but any other suitable host cell may be used as would be understood by a person of skill in the art. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can subsequently be recovered from the culture medium using standard protein purification methods.

Yeast systems which may be used include *Pichia pastoris, Kluyveromyces lactis* and *Saccharomyces cerevisiae.* Bacterial systems which would be appropriate for this purpose may include *Escherichia coli, Corynebacterium,* and *Pseudomonas fluorescens.* A cell free expression system may also be utilised wherein protein production is performed *in vitro,* using purified RNA polymerase, ribosomes, tRNA and ribonucleotides.

In yet a further aspect the bispecific binding protein herein disclosed is intended for use as a therapy in subjects, preferably wherein the subject has cancer or an infectious disease.

The terms 'cancer' and 'tumour' are used interchangeably and refer to a cell or population of cells whose growth, proliferation or survival is greater than growth, proliferation or survival of a normal counterpart cell, e.g. a cell proliferative or differentiative disorder. Typically, the growth is uncontrolled. The term 'malignancy' refers to the invasion of nearby tissue by the cancerous cells.

The term 'infectious disease' refers to a group of disorders caused by microorganisms; bacteria, viruses, fungi or parasites. The diseases caused by each may be spread directly or indirectly.

The term 'therapy' or 'treatment' refers to administering an active agent with the purpose to cure, heal, alleviate, relieve, alter, remedy, ameliorate, improve or affect a condition (e.g. a disease), the symptoms of the condition, or to prevent or delay the onset of symptoms, complications, biochemical indicia of a disease, or otherwise arrest or inhibit further development of the disease, condition, or disorder in a statistically significant way.

The present invention provides a pharmaceutical composition comprising the bispecific binding protein, nucleic acid, vector or host cell of the preceding aspects and embodiments at a therapeutically effective amount in combination with a pharmaceutically acceptable carrier and/or a known therapeutic agent.

The term 'therapeutically effective amount' refers to the amount of the bispecific binding protein that preferably results in a decrease in severity of disease symptoms, an increase in frequency and duration of disease symptom-free period, or a prevention of impairment or disability due to the disease affliction (i.e. cancer or infectious disease). The term 'effect amount' refers to the sufficient amount of an agent required to provide the desired biological or therapeutic result. That result can be reduction, amelioration, palliation, lessening, delaying, and/or alleviation of one or more of the signs, symptoms or causes of a disease or any other desired alteration of a biological system. In reference to cancer, an effective amount may comprise an amount sufficient to cause a tumour to shrink and/or to decrease the growth rate of the tumour (such as to suppress tumour growth) or to prevent or delay other unwanted cell proliferation. In some embodiments, an effective amount sufficient to delay development, or prolong survival or induce stabilisation of the cancer or tumour. In reference to infectious disease, an effective amount may comprise a reduction in viral load and or an increase in the subjects CD4 count (a measure indicative of the strength of the immune system).

More than one pharmaceutically acceptable carrier and therapeutic agent may be included in the composition. Additionally, two or more bispecific binding proteins may be included in the pharmaceutical composition, each of which may target a different immune checkpoint molecule, for example, PD-1 and CTLA-4, PD-1 and CD47, PD-L1 and CTLA-4, PD-L1 and CD47, PD-L1 and PD-1 or CTLA-4 and CD47.

A pharmaceutically acceptable carrier is intended to refer to any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration. Depending on the route of administration, the administered product (i.e. the bispecific binding protein or the vector as a delivery system) may be coated in a material to protect the product from adverse physiological conditions that may result in inactivation of the product (for example, high acidity).

The pharmaceutical composition may comprise a pharmaceutically acceptable salt; this term refers to a salt that retains the desired biological activity of the parent compound and does not impart any undesired toxicological effects. Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N'-dibenzylethylenediamine, N-methylglucamine, chloroprocaine, choline, diethanolamine, ethylenediamine, procaine and the like.

A pharmaceutical composition of the disclosure also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and non-aqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

The pharmaceutical compositions may further contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The present invention includes a pharmaceutical composition which may optionally include an additional therapeutic agent, known to be efficacious in, for example, cancer. For example, the present invention may be used in combination with known chemotherapeutic agents, for example, altretamine, busulfan, carboplatin, carmustin, cisplatin and decarbazine, as a method to reduce their levels of toxicity. The present invention may also be used in combination with infectious disease therapeutics, for example, antiretroviral therapy, antibiotics, antifungals and antiparasitics. The pharmaceutical composition may be given concurrently to additional therapies, for example, radiotherapy or chemotherapy for subjects with cancer. The term 'concurrently' refers to administration of said therapies occurring on the same day.

The present invention may also include the presence of one or more immunogenic agents. By 'immunogenic agents' we intend any molecule that is capable of inducing an immune response. Examples of such immunogenic agents include cancerous cells, purified tumour antigen and cells transfected with genes encoding immune stimulating cytokines. The use of these agents in combination with the bispecific binding protein disclosed herein may be used to generate an enhanced immune response against a target, for example, tumour cells.

The present invention is intended for use in the treatment of cancer, preferably wherein the cancer is a solid tumour. By 'solid tumour' we intend an abnormal mass of tissue that typically lacks cysts or areas of liquid. It is intended that cancers that are particularly responsive to immunotherapy intervention may be targeted. Accordingly, subject samples may be collected to determine expression levels of, for example, PD-L1 on tumour cells to determine if a bispecific binding protein directed to PD-1 would be efficacious. Examples of cancers which may be treated according to the present invention include, but are not limited to: melanoma, renal cancer, prostate cancer, breast cancer, colon cancer, lung cancer, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the oesophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukaemia's including acute myeloid leukaemia, chronic myeloid leukemia, acute lymphoblastic leukemia, chronic lymphocytic leukemia, solid tumours of childhood, lymphocytic lymphoma, cancer of the bladder, cancer of the kidney or ureter, carcinoma of the renal pelvis, neoplasm of the central nervous system (CNS), primary CNS lymphoma, tumour angiogenesis, spinal axis tumour, brain stem glioma, pituitary adenoma, Kaposi's sarcoma, epidermoid cancer, squamous cell cancer, T-cell lymphoma, and combinations of said cancers. The present invention is also suitable for use in metastatic cancers, particularly those which are known to express PD-1, PD-L1, CD47 and CTLA-4. It is envisaged that the bispecific binding protein herein disclosed may be of particular use in cancers of the CNS owing to its smaller size, and therefore its potential to be engineered to cross the blood-brain barrier, compared to antibody molecules of a larger size, for example, IgGs.

In yet a further aspect, the present invention is intended for use in infectious disease, wherein the infectious disease may be a viral infection, a bacterial infection, a parasitic infection or a fungal infection. Examples of possible viral infections to be treated include human immunodeficiency virus (HIV), ebola virus, hepatitis virus (A, B, or C), herpes virus (e.g, VZV, HSV-I, HAV-6, HSV-II, and CMV, Epstein Barr virus), adenovirus, influenza virus, flaviviruses, echovirus, rhinovirus, coxsackie virus, coronavirus, respiratory syncytial virus, mumps virus, rotavirus, measles virus, rubella virus, parvovirus, vaccinia virus, HTLV virus, dengue virus, papillomavirus, molluscum virus, poliovirus, rabies virus, JC virus or arboviral encephalitis virus. Examples of possible bacterial infections include *Chlamydia,* rickettsial bacteria, mycobacteria, staphylococci, streptococci, pneumonococci, meningococci and gonococci, klebsiella, proteus, serratia, pseudomonas, *Legionella, Corynebacterium diphtheriae, Salmonella,* bacilli, *Vibrio cholerae, Clostridium tetan, Clostridium botulinum, Bacillus anthricis, Yersiniapestis, Mycobacterium leprae, Mycobacterium lepromatosis, and Borriella.* Examples of possible parasitic infections to be treated include *Entamoeba histolytica, Balantidium coli, Naegleria fowleri, Acanthamoeba, Giardia lambia, Cryptosporidium, Pneumocystis carinii, Plasmodium vivax, Babesia microti, Trypanosoma brucei, Trypanosoma cruzi, Leishmania donovani, Toxoplasma gondii, Nippostrongylus brasiliensis.* Examples of possible fungal infections to be treated include *Candida (albicans, krusei, glabrata, tropicalis, etc.), Cryptococcus neoformans, Aspergillus (fumigatus, niger, etc.),* Genus *Mucorales (mucor, absidia, rhizopus), Sporothrix schenkii, Blastomyces dermatitidis, Paracoccidioides brasiliensis, Coccidioides immitis and Histoplasma capsulatum.*

The invention will now be illustrated in the following examples with reference to the accompanying drawings.

### Examples

### Example 1

The inventors have previously identified lead single domain antibody clones against PD-1. These clones are referred to as 9E3 and 3H4. The 3H4 clone is known to have a lower blocking capability compared to 9E3. When an *in vitro* ELISA-based blocking assay was performed with the two clones given in combination, it was found that a significantly lower blocking capability was achieved as compared to the commercially available Nivolumab (a PD-1 blocking antibody).

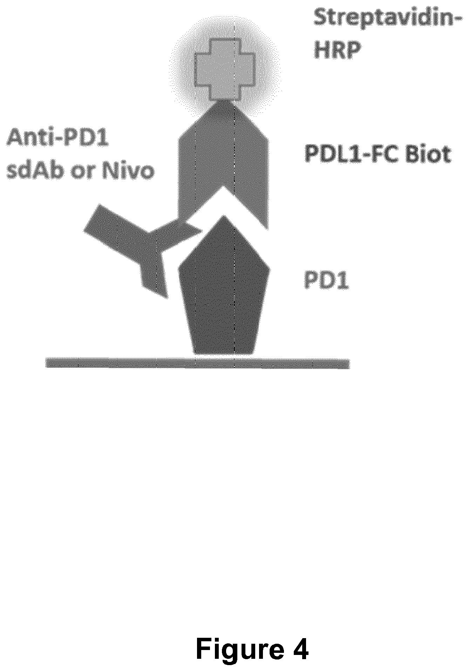

Briefly, the blocking capabilities of the individual clones were assessed by ELISA using Recombinant Human PD-1 (R&D Systems Inc) bound to the surface (see Figure 4). The binding interaction between PD-1 and a biotinlylated recombinant human PD-L1Fc (R&D Systems Inc) was then disrupted by introducing a range of concentrations of the blocking antibodies or Nivolumab. The disruption of the binding signal was then detected using streptavidin conjugated to a horseradish peroxidase detection enzyme along with an appropriate substrate.

Novel bispecific single domain antibodies were subsequently generated using the 9E3 and 3H4 PD-1 lead blocking clones, connected by a peptide linker. This peptide linker varied in length from 17 amino acid residues (17 aa) to 30 amino acid residues (30 aa) in length. Additionally, the orientation of the two lead clones was alternated for each peptide linker length. Four different constructs were thus generated for testing; i) 9E3-30aa-3H4, ii) 9E3-17aa-3H4, iii) 3H4-30aa-9E3 and iv) 3H4-17aa-9E3. These constructs were compared against the individual effect of the 9E3 and 3H4 clones, and against Nivolumab.

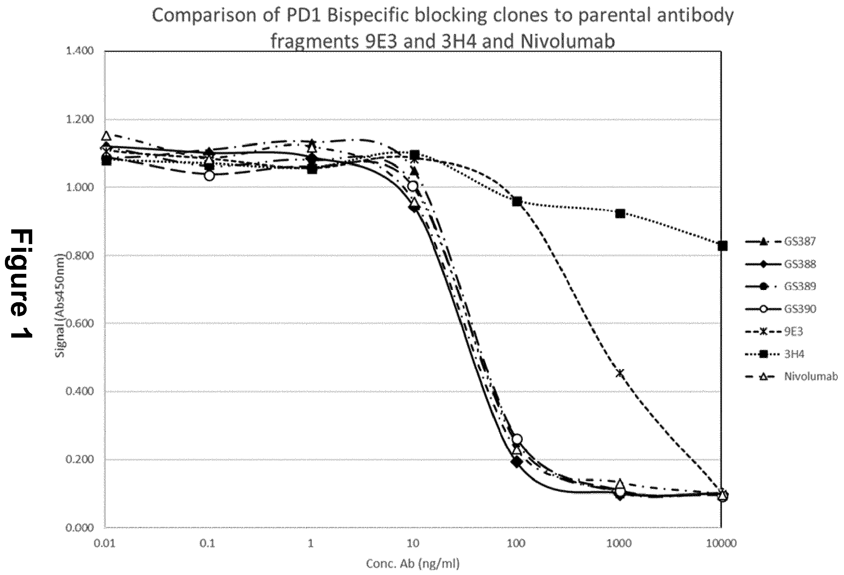

Surprisingly, it was found that all four constructs showed blocking capability approximately equal to that of Nivolumab, with the 9E3-17aa-3H4 construct, also known as GS388, (SEQ ID NO: 1) demonstrating slightly higher blocking activity than the rest of the constructs as well as Nivolumab (Figure 1 and Table 1). The present invention therefore may achieve at least the same blocking capability as a commercially available antibody against PD-1 with reduced toxicity.

**Table 1: Binding capability of GS388, 9E3, 3H4 and Nivolumab at a range of different concentrations.**

| Ab Conc. | Clone ID | | | |
|---|---|---|---|---|
| (ng/ml) | GS388 Bi | 9E3 | 3H4 | Nivolumab |
| 10000 | 0.102 | 0.104 | 0.833 | 0.100 |
| 1000 | 0.103 | 0.457 | 0.927 | 0.134 |
| 100 | 0.197 | 0.965 | 0.964 | 0.234 |
| 10 | 0.947 | 1.087 | 1.100 | 0.963 |
| 1 | 1.088 | 1.057 | 1.058 | 1.120 |
| 0.1 | 1.100 | 1.085 | 1.071 | 1.087 |
| 0.01 | 1.119 | 1.110 | 1.083 | 1.155 |
| 0.001 | 1.113 | 1.173 | 1.114 | 1.159 |

### Example 2

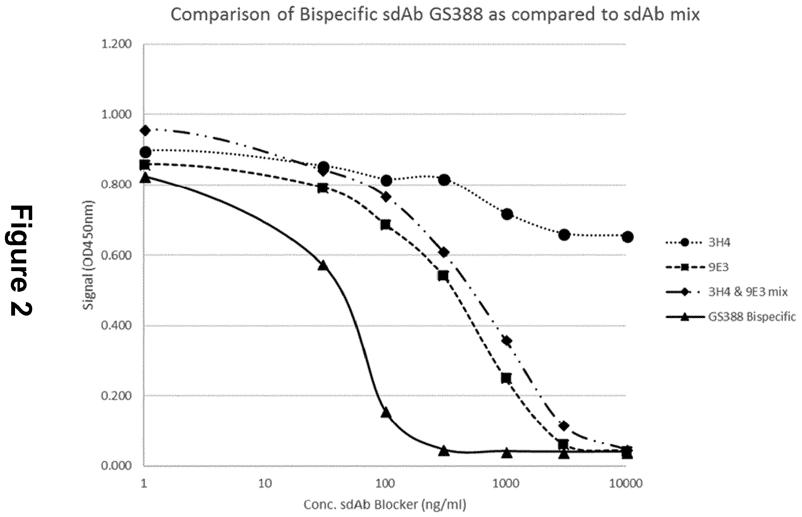

A second blocking assay was performed in a similar assay format to above comparing the lead bispecific antibody GS388 to a simple mix of its constituent sdAbs 3H4 and 9E3. The results showed >10 fold increase in blocking by the bispecific clone as compared to a simple mix of the constituent antibodies 9E3 and 3H4 alone (See Figure 2). This indicates that physical linkage of the two antibody constituents provides a very significant advantage as compared to simply combining the constituent antibodies in a mixture with no physical linkage.

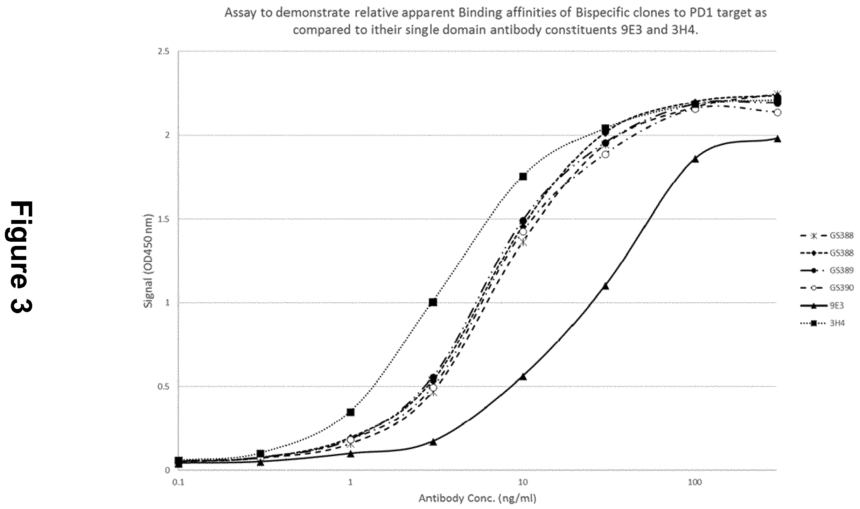

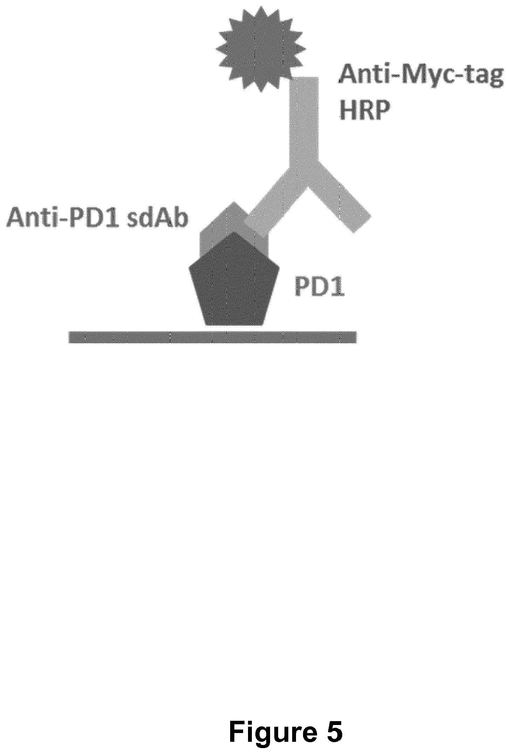

A further ELISA based assay was subsequently carried out to compare the apparent affinities of GS388 and its parental sdAb constituent clones (See Figure 5). In brief, PD1 antigen was coated on an ELISA surface prior to blocking of the surface and probing with a gradient of either of the two constituent single domain antibodies 9E3 and 3H4 or one of the four bispecific derivatives, followed by washing and detection with an anti cMyc-tag HRP detector conjugate. The results of this assay demonstrated no increase in the apparent affinity of the bispecific clone as compared to that of the individual sdAb parents. Instead it could be seen that the four bispecific clones had similar IC50 values which were intermediate to those of the two constituent fragments 9E3 and 3H4 (See Figure 3).

The inventors have therefore surprisingly demonstrated there to be no increase in relative apparent affinity for the bispecific blocking clone GS388 as compared to that of its individual sdAb parents 9E3 and 3H4. This suggests that the increased blocking effect of the bispecific clone GS388 is due not to an increase in avidity from linking the two clones together as described for a different purpose in Jähnichen et al (2010) but rather is due to a physical blockage or steric hindrance of the receptor binding site caused by the peptide linker which links the two sdAb domains together crossing over the surface of the receptor binding site.

### Sequences used throughout the specification and forming part of the description:

**SEQ ID NO: 1 (Bispecific clone GS388)** 
QLQLVEFGGGLVQAGGSLRLSCAASGSIFRGNAMVWYRQAPGKERELVATIT
NGGSTNYADSVKGRFTISRDNAKNTVYLQMNSLKPEDTAIYYCNDVIRGESGIY
YPLNYWGQGTQVTVSSGGSEGGGSGGGGESGGGQVQFVESGGGLVQPGG
SLRLSCAVSGSTFVVKHFAWFRQPPGNRRELVAATRGEGTTYYRDSVMGRFT
ISSDNPENTVYLQMTSLQPEDTAVYFCNVRSPGGEEFWGKGTLVTVSS
**SEQ ID NO: 2 (9E3 single domain antibody)** 
QLQLVEFGGGLVQAGGSLRLSCAASGSIFRGNAMVWYRQAPGKERELVATIT
NGGSTNYADSVKGRFTISRDNAKNTVYLQMNSLKPEDTAIYYCNDVIRGESGIY
YPLNYWGQGTQVTVSS
**SEQ ID NO: 3 (3H4 single domain antibody)** 
QVQFVESGGGLVQPGGSLRLSCAVSGSTFVVKHFAWFRQPPGNRRELVAAT
RGEGTTYYRDSVMGRFTISSDNPENTVYLQMTSLQPEDTAVYFCNVRSPGGE
EFWGKGTLVTVSS
**SEQ ID NO: 4 (9E3 CDR region 1)**
   SIFRGNA
**SEQ ID NO: 5 (9E3 CDR region 2)**
   TITNGGSTN
**SEQ ID NO: 6 (9E3 CDR region 3)**
   NDVIRGESGIYYPLNY
**SEQ ID NO: 7 (3H4 CDR region 1)**
   STFVVKH
**SEQ ID NO: 8 (3H4 CDR region 2)**
   ATRGEGTTY
**SEQ ID NO: 9 (3H4 CDR region 3)**
   NVRSPGGEEF
**SEQ ID NO: 10 (17 amino acid peptide linker)**
   GGSEGGGSGGGGESGGG
**SEQ ID NO: 11 (Bispecific IgG1 molecule)** 
QLQLVEFGGGLVQAGGSLRLSCAASGSIFRGNAMVWYRQAPGKERELVATIT
NGGSTNYADSVKGRFTISRDNAKNTVYLQMNSLKPEDTAIYYCNDVIRGESGIY
YPLNYWGQGTQVTVSSGGSEGGGSGGGGESGGGQVQFVESGGGLVQPGG
SLRLSCAVSGSTFVVKHFAWFRQPPGNRRELVAATRGEGTTYYRDSVMGRFT
ISSDNPENTVYLQMTSLQPEDTAVYFCNVRSPGGEEFWGKGTLVTVSSepkssd




**SEQ ID NO: 12 (Fc fusion tag)**

epksssdkthtcppcpapellggpsvflfppkpkdtlmisrtpevtcvvvdvshedpevkfnwyvdgvevhnak
tkpreeqynstyrvvsvltvlhqdwlngkeykckvsnkalpapiektiskakgqprepqvytlppsrdeltknqvs
ltclvkgfypsdiavewesngqpennykttppvldsdgsfflyskltvdksrwqqgnvfscsvmhealhnhytq
kslslspgk

## Claims

1. A bispecific binding protein comprising a first functional moiety and a second functional moiety each having binding affinity for an immune checkpoint molecule, wherein the first functional moiety and the second functional moiety are connected, and wherein the first functional moiety and the second functional moiety bind to different discrete epitopes on the surface of the same immune checkpoint molecule.

2. The bispecific binding protein of claim 1, wherein binding affinity is for the immune checkpoint molecule PD-1, PD-L1, LAG-3, CD47 or CTLA-4, preferably wherein the immune checkpoint molecule is PD-1.

3. The bispecific binding protein of any preceding claim, wherein the first functional moiety and the second functional moiety are connected by a peptide linker, preferably wherein the linker is 5-100 amino acid residues in length, preferably 8 to 30 amino acids residues in length, more preferably 10 to 17 amino acid residues in length.

4. The peptide linker of claim 3, wherein the peptide linker comprises the amino acid sequence of SEQ ID NO: 10 or an amino acid sequence having greater than 60 % homology to said amino acid sequence.

5. The bispecific binding protein of claims 1 to 4, wherein the first functional moiety and the second functional moiety are connected by paired fusion tags, preferably wherein the paired fusion tags are a leucine zipper or an immunoglobulin Fc tag.

6. The bispecific binding protein of any preceding claim, wherein the bispecific binding protein is a bispecific antibody or a functional fragment thereof.

7. The bispecific binding protein of claim 6, wherein the first functional moiety and the second functional moiety comprise a fragment antigen binding (Fab) fragment, a single-chain variable fragment (scFv), an affimer or equivalent protein-binding protein, or a single-domain antibody (sdAb), preferably wherein the first functional moiety and the second functional moiety are each a single-domain antibody.

8. The bispecific binding protein of claims 1-5, wherein the bispecific binding protein further comprises an Fc fusion tag comprising the amino acid sequence of SEQ ID NO: 12 or an amino acid sequence having greater than 60 % homology to said amino acid.

9. The bispecific binding protein of any preceding claim comprising the amino acid sequence of SEQ ID NO: 1 or SEQ ID NO: 11 or an amino acid sequence having greater than 60 % homology to said amino acid sequence.

10. The bispecific binding protein of any preceding claim, wherein the first functional moiety comprises the amino acid sequence of SEQ ID NO: 2 and the second functional moiety comprises the amino acid sequence of SEQ ID NO: 3, or an amino acid sequence having greater than 60 % homology to said amino acid sequences.

11. The bispecific binding protein of any preceding claim, wherein the first functional moiety comprises the CDR amino acid sequence of SEQ ID NO: 4, SEQ ID NO: 5 and SEQ ID NO: 6 and the second functional moiety comprises the CDR amino acid sequence of SEQ ID NO: 7, SEQ ID NO: 8, SEQ ID NO: 9 or an amino acid sequence having greater than 60 % homology to said amino acid sequences.

12. A nucleic acid encoding the bispecific binding protein of claims 1-11, or a complementary sequence thereof.

13. An expression vector comprising the nucleic acid of claim 12, or a host cell or cell free expression system comprising said expression vector.

14. The bispecific binding protein according to any of claims 1-11, for use in therapy.

15. A pharmaceutical composition comprising the bispecific binding protein, nucleic acid, vector or host cell according to any one of claims 1-14, wherein the composition additionally includes a pharmaceutically acceptable carrier and/or a known therapeutic agent.

16. The pharmaceutical composition of claim 15, wherein the bispecific binding protein is encoded in a viral vector, preferably wherein the viral vector is an oncolytic virus and intended for *in vivo* expression in a subject.

17. The pharmaceutical composition of claims 15-16 for use in the treatment of cancer, preferably wherein the cancer is a solid tumour, or for use in the treatment of infectious disease.


SEQUENCE LISTING

<110>  RANDOX LABORATORIES LTD.

<120>  IMMUNE CHECKPOINT MOLECULE INHIBITORS

<130>  JWJ02542EP

<140>
<141>

<160>  12

<170>  PatentIn version 3.5

<210>  1
<211>  255
<212>  PRT
<213>  Artificial Sequence

<220>
<223>  Bispecific clone GS388

<400>  1

Gln Leu Gln Leu Val Glu Phe Gly Gly Gly Leu Val Gln Ala Gly Gly
1               5                   10                  15

Ser Leu Arg Leu Ser Cys Ala Ala Ser Gly Ser Ile Phe Arg Gly Asn
            20                  25                  30

Ala Met Val Trp Tyr Arg Gln Ala Pro Gly Lys Glu Arg Glu Leu Val
        35                  40                  45

Ala Thr Ile Thr Asn Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Ile Tyr Tyr Cys Asn
                85                  90                  95

Asp Val Ile Arg Gly Glu Ser Gly Ile Tyr Tyr Pro Leu Asn Tyr Trp
            100                 105                 110

Gly Gln Gly Thr Gln Val Thr Val Ser Ser Gly Gly Ser Glu Gly Gly
        115                 120                 125

Gly Ser Gly Gly Gly Gly Glu Ser Gly Gly Gly Gln Val Gln Phe Val
    130                 135                 140

Glu Ser Gly Gly Gly Leu Val Gln Pro Gly Gly Ser Leu Arg Leu Ser
145                 150                 155                 160

Cys Ala Val Ser Gly Ser Thr Phe Val Val Lys His Phe Ala Trp Phe
                165                 170                 175

Arg Gln Pro Pro Gly Asn Arg Arg Glu Leu Val Ala Ala Thr Arg Gly
            180                 185                 190

Glu Gly Thr Thr Tyr Tyr Arg Asp Ser Val Met Gly Arg Phe Thr Ile
        195                 200                 205

Ser Ser Asp Asn Pro Glu Asn Thr Val Tyr Leu Gln Met Thr Ser Leu
    210                 215                 220

Gln Pro Glu Asp Thr Ala Val Tyr Phe Cys Asn Val Arg Ser Pro Gly
225                 230                 235                 240

Gly Glu Glu Phe Trp Gly Lys Gly Thr Leu Val Thr Val Ser Ser
                245                 250                 255

<210>  2
<211>  122
<212>  PRT
<213>  Artificial Sequence

<220>
<223>  9E3 single domain antibody

<400>  2

Gln Leu Gln Leu Val Glu Phe Gly Gly Gly Leu Val Gln Ala Gly Gly
1               5                   10                  15

Ser Leu Arg Leu Ser Cys Ala Ala Ser Gly Ser Ile Phe Arg Gly Asn
            20                  25                  30

Ala Met Val Trp Tyr Arg Gln Ala Pro Gly Lys Glu Arg Glu Leu Val
        35                  40                  45

Ala Thr Ile Thr Asn Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Ile Tyr Tyr Cys Asn
                85                  90                  95

Asp Val Ile Arg Gly Glu Ser Gly Ile Tyr Tyr Pro Leu Asn Tyr Trp
            100                 105                 110

Gly Gln Gly Thr Gln Val Thr Val Ser Ser
        115                 120

<210>  3
<211>  116
<212>  PRT
<213>  Artificial Sequence

<220>
<223>  3H4 single domain antibody

<400>  3

Gln Val Gln Phe Val Glu Ser Gly Gly Gly Leu Val Gln Pro Gly Gly
1               5                   10                  15

Ser Leu Arg Leu Ser Cys Ala Val Ser Gly Ser Thr Phe Val Val Lys
            20                  25                  30

His Phe Ala Trp Phe Arg Gln Pro Pro Gly Asn Arg Arg Glu Leu Val
        35                  40                  45

Ala Ala Thr Arg Gly Glu Gly Thr Thr Tyr Tyr Arg Asp Ser Val Met
    50                  55                  60

Gly Arg Phe Thr Ile Ser Ser Asp Asn Pro Glu Asn Thr Val Tyr Leu
65                  70                  75                  80

Gln Met Thr Ser Leu Gln Pro Glu Asp Thr Ala Val Tyr Phe Cys Asn
                85                  90                  95

Val Arg Ser Pro Gly Gly Glu Glu Phe Trp Gly Lys Gly Thr Leu Val
            100                 105                 110

Thr Val Ser Ser
        115

<210>  4
<211>  7
<212>  PRT
<213>  Artificial Sequence

<220>
<223>  9E3 CDR region 1

<400>  4

Ser Ile Phe Arg Gly Asn Ala
1               5

<210>  5
<211>  9

<212>  PRT
<213>  Artificial Sequence

<220>
<223>  9E3 CDR region 2

<400>  5

Thr Ile Thr Asn Gly Gly Ser Thr Asn
1               5


<210>  6
<211>  16
<212>  PRT
<213>  Artificial Sequence

<220>
<223>  9E3 CDR region 3

<400>  6

Asn Asp Val Ile Arg Gly Glu Ser Gly Ile Tyr Tyr Pro Leu Asn Tyr
1               5                   10                  15


<210>  7
<211>  7
<212>  PRT
<213>  Artificial Sequence

<220>
<223>  3H4 CDR region 1

<400>  7

Ser Thr Phe Val Val Lys His
1               5


<210>  8
<211>  9
<212>  PRT
<213>  Artificial Sequence

<220>
<223>  3H4 CDR region 2

<400>  8

Ala Thr Arg Gly Glu Gly Thr Thr Tyr
1               5


<210>  9
<211>  10
<212>  PRT
<213>  Artificial Sequence

<220>
<223>  3H4 CDR region 3

<400>  9

Asn Val Arg Ser Pro Gly Gly Glu Glu Phe
1               5                   10

<210>  10
<211>  17
<212>  PRT
<213>  Artificial Sequence

<220>
<223>  17 amino acid peptide linker

<400>  10

Gly Gly Ser Glu Gly Gly Gly Ser Gly Gly Gly Gly Glu Ser Gly Gly
1               5                   10                  15

Gly

<210>  11
<211>  487
<212>  PRT
<213>  Artificial Sequence

<220>
<223>  Bispecific IgG1 molecule

<400>  11

Gln Leu Gln Leu Val Glu Phe Gly Gly Gly Leu Val Gln Ala Gly Gly
1               5                   10                  15

Ser Leu Arg Leu Ser Cys Ala Ala Ser Gly Ser Ile Phe Arg Gly Asn
            20                  25                  30

Ala Met Val Trp Tyr Arg Gln Ala Pro Gly Lys Glu Arg Glu Leu Val
        35                  40                  45

Ala Thr Ile Thr Asn Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Ile Tyr Tyr Cys Asn
                85                  90                  95

Asp Val Ile Arg Gly Glu Ser Gly Ile Tyr Tyr Pro Leu Asn Tyr Trp
            100                 105                 110

Gly Gln Gly Thr Gln Val Thr Val Ser Ser Gly Gly Ser Glu Gly Gly
        115                 120                 125

Gly Ser Gly Gly Gly Gly Glu Ser Gly Gly Gly Gln Val Gln Phe Val
    130                 135                 140

Glu Ser Gly Gly Gly Leu Val Gln Pro Gly Gly Ser Leu Arg Leu Ser
145                 150                 155                 160

Cys Ala Val Ser Gly Ser Thr Phe Val Val Lys His Phe Ala Trp Phe
                165                 170                 175

Arg Gln Pro Pro Gly Asn Arg Arg Glu Leu Val Ala Ala Thr Arg Gly
            180                 185                 190

Glu Gly Thr Thr Tyr Tyr Arg Asp Ser Val Met Gly Arg Phe Thr Ile
        195                 200                 205

Ser Ser Asp Asn Pro Glu Asn Thr Val Tyr Leu Gln Met Thr Ser Leu
    210                 215                 220

Gln Pro Glu Asp Thr Ala Val Tyr Phe Cys Asn Val Arg Ser Pro Gly
225                 230                 235                 240

Gly Glu Glu Phe Trp Gly Lys Gly Thr Leu Val Thr Val Ser Ser Glu
                245                 250                 255

Pro Lys Ser Ser Asp Lys Thr His Thr Cys Pro Pro Cys Pro Ala Pro
            260                 265                 270

Glu Leu Leu Gly Gly Pro Ser Val Phe Leu Phe Pro Pro Lys Pro Lys
        275                 280                 285

Asp Thr Leu Met Ile Ser Arg Thr Pro Glu Val Thr Cys Val Val Val
    290                 295                 300

Asp Val Ser His Glu Asp Pro Glu Val Lys Phe Asn Trp Tyr Val Asp
305                 310                 315                 320

Gly Val Glu Val His Asn Ala Lys Thr Lys Pro Arg Glu Glu Gln Tyr
                325                 330                 335

Asn Ser Thr Tyr Arg Val Val Ser Val Leu Thr Val Leu His Gln Asp
            340                 345                 350

Trp Leu Asn Gly Lys Glu Tyr Lys Cys Lys Val Ser Asn Lys Ala Leu
        355                 360                 365

Pro Ala Pro Ile Glu Lys Thr Ile Ser Lys Ala Lys Gly Gln Pro Arg

370                 375                 380

Glu Pro Gln Val Tyr Thr Leu Pro Pro Ser Arg Asp Glu Leu Thr Lys
385                 390                 395                 400

Asn Gln Val Ser Leu Thr Cys Leu Val Lys Gly Phe Tyr Pro Ser Asp
                405                 410                 415

Ile Ala Val Glu Trp Glu Ser Asn Gly Gln Pro Glu Asn Asn Tyr Lys
            420                 425                 430

Thr Thr Pro Pro Val Leu Asp Ser Asp Gly Ser Phe Phe Leu Tyr Ser
        435                 440                 445

Lys Leu Thr Val Asp Lys Ser Arg Trp Gln Gln Gly Asn Val Phe Ser
    450                 455                 460

Cys Ser Val Met His Glu Ala Leu His Asn His Tyr Thr Gln Lys Ser
465                 470                 475                 480

Leu Ser Leu Ser Pro Gly Lys
                485

<210>  12
<211>  232
<212>  PRT
<213>  Artificial Sequence

<220>
<223>  Fc fusion tag

<400>  12

Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230